# EUROPEAN PATENT SPECIFICATION

(11) **EP 3 367 947 B1**
(45) Date of publication and mention of the grant of the patent: **16.06.2021**
(21) Application number: 16860595.4
(22) Date of filing: 25.10.2016
(51) Int. Cl.: A61B 17/34

(54) **NERVE AND SOFT TISSUE SURGICAL DEVICE**
NERVEN- UND WEICHTEILCHIRURGIEVORRICHTUNG
DISPOSITIF CHIRURGICAL D'ABLATION DE TISSUS NERVEUX ET MOUS

(30) Priority: 28.10.2015 US 201514924885
(43) Date of publication of application: 05.09.2018
(73) Proprietor: Warsaw Orthopedic, Inc., Warsaw, IN 46582 (US)
(72) Inventor: MCKAY, William F., Memphis, Tennessee 38133 (US)
(74) Representative: Viering, Jentschura & Partner mbB Patent- und Rechtsanwälte
(86) International application number: PCT/US2016/058590
(87) International publication number: WO 2017/074901

(56) References cited:
- JP-A- H05 192 345
- US-A1- 2005 251 124
- US-A1- 2007 106 176
- US-A1- 2008 051 776
- US-A1- 2010 168 675
- US-A1- 2013 310 751
- US-A1- 2014 276 701
- US-A1- 2014 276 701
- US-B1- 6 494 844
- US-B1- 6 494 844

## Description

### FIELD

The present application relates generally to devices for ablating a material or substance. More specifically, the devices are useful for removing nerve and/or soft tissue via a minimally invasive procedure to alleviate pain.

### BACKGROUND

Acute and chronic pain management has been a concern for as long as medicine has been practiced. Several methods of inducing analgesia and anesthesia have been developed. For example, the use of chemical substances is perhaps the most common approach to pain relief which requires suitable substances that are effective, safe to humans, and do not cause complications or abnormal reactions. Despite the great advances that have been made in the field of anesthesiology and in the field of pain relief in general, there are still some drawbacks to chemical-based approaches. For instance, the anesthetics generally available today must be administered in carefully graduated doses to assure the patient's well-being, require extended periods of fasting prior to treatment, and are often accompanied by undesirable after effects such as nausea.

One alternative approach that is commonly used for providing pain relief is ablation, in which nerves and/or tissue is removed and/or destroyed. Two approaches to removing tissue via ablation are through cold or hot ablation procedures and techniques. Various categories of ablation include but are not limited to electrical, radiation, light, radiofrequency, ultrasound, cryotherapy, thermal, microwave and hydromechanical. One form of hot ablation is radiofrequency (RF) ablation. During RF ablation, current passing through tissue from the active electrode leads to ion agitation, which is converted by means of friction into heat. The process of cellular heating includes almost immediate and irreparable cellular damage, which leads to coagulation necrosis. Because ion agitation, and thus tissue heating, is greatest in areas of highest current density (e.g., closest to the active electrode tip), necrosis is limited to a relatively small volume of tissue surrounding the RF electrode.

Another form of ablation uses cold ablation and is called cryoablation. During cryoablation, tissue is frozen or rapid freeze/thaw cycles are inflicted upon the tissue. There are many advantages to using cryoablation instead of radiofrequency ablation. For example, cryoablation is safer especially near critical vasculature and there is less risk of post-procedure neuritis or neuromas following neuroablation for the treatment of pain. Cryoablation allows treatment mapping pre and post procedure where areas of tissue can be mapped by limited, reversible and/or freezing. Cryoablation can be monitored and visualized on ultrasonography, CT and MRI. Moreover, because nerve cooling is anesthetic, cryoablation is a less painful procedure than thermal ablation techniques.

US 6494844 B1 discloses a system for treating or sampling of a mass within the breast of a human patient, wherein said system comprises a cannula adapted to apply suction through the lumen of the catheter to the tumor or lesion; and wherein the lumen has a self-sealing valve through which a cryoprobe is inserted while the suction is being applied. JP H05192345 A discloses a surgical apparatus comprising a horn for crushing a tissue by ultrasonic oscillation generated by an ultrasonic oscillator, and an electrode for cutting and coagulating the tissue by high-frequency current, wherein the horn and the electrode are placed in the same handpiece.

Traditional cryoablation systems can provide removal capabilities of soft tissue via the application of single needles that form an ice ball centered around a tip, but because the needles are not insulated, the procedures cause an increase in heat loss for adjacent tissues at a surgical site. The heat loss will sometimes lead to tissue freezing and potential necrosis. In addition, there is an increase in radiation exposure to the surgical site. One particularly effective form of insulation for cryoablation is the presence of an air pocket immediately surrounding the cryoablation probe.

Cryoablation systems often require being passed through some depth of tissue to ablate a selected region of deep tissue. To access the deep tissue, a medical practitioner may use an insertion cannula or needle to create a surgical pathway to the selected region. However, such surgical pathways are not liquid proof and fail to provide a means for maintaining a uniform air pocket on all sides of the cryoablation probe. The presence of such liquid in the surgical pathway and an uneven air pocket surrounding the cryoablation probe disrupt the insulation of the probe, potentially causing unwanted damage to the surrounding tissue.

Accordingly, there is a need for devices and methods to provide a surgical pathway to insulate a cryoablation probe to prevent unwanted damage to tissue. There is a need for an introducer needle which facilitates the creation of a uniform air gap surrounding a cryoablation probe to provide insulation. There is also a need for an introducer which provides a fluid proof seal to prevent entrance of fluids into the surgical pathway. There is also a need for an introducer which removes fluid from a cryoablation probe or other surgical tools which are inserted into the surgical pathway. Moreover, a device is needed for use during a minimally invasive procedure and/or during an open surgical procedure. Further, there is a need for devices and methods that provide fine ablation capabilities of nerve and/or soft tissue.

### SUMMARY

The present invention discloses a surgical device according to claim 1, and a surgical system according to claim 8. Further embodiments of the present invention are disclosed in the dependent claims. Ablation devices and exemplary methods not forming part of the invention are provided that insulate a cryoablation probe to prevent unwanted damage to tissue. Devices and exemplary methods not forming part of the invention are also provided for an insertion cannula or introducer needle which facilitates the creation of a uniform air gap surrounding a cryoablation probe to provide insulation. Devices and exemplary methods not forming part of the invention are also provided for an insertion cannula or introducer which provides a fluid proof seal to prevent entrance of fluids into the surgical pathway. Devices and exemplary methods not forming part of the invention are also provided for an introducer or insertion cannula, which removes fluid from a cryoablation probe or other surgical tools which are inserted into the surgical pathway. Moreover, a device is provided for use during a minimally invasive procedure and/or during an open surgical procedure. Further, provided are devices and exemplary methods not forming part of the invention that provide fine ablation capabilities of nerve and/or soft tissue.

Devices and exemplary methods not forming part of the invention of use for an insertion cannula or introducer cannula to insulate a cryoablation probe or other surgical devices are provided.

In some examples, exemplary methods not forming part of the invention of ablating a nerve and/or soft tissue are provided. In some examples, a method comprises disposing an insertion cannula at a surgical site, the insertion cannula having a proximal end, an exterior surface comprising a distal tip configured to penetrate tissue, and an interior surface that defines an internal passage of the insertion cannula, the internal passage extending along a longitudinal axis of the insertion cannula and configured to receive an ablation probe, a sealing member contacting the proximal end of the insertion cannula and configured to provide a fluid seal to prevent fluid from entering or leaving the proximal end of the insertion cannula; and passing a surgical tool through the internal passage of the insertion cannula to the nerve and/or soft tissue to ablate the nerve and/or soft tissue.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE DRAWINGS

In part, other aspects, features, benefits and advantages of the embodiments will be apparent with regard to the following description, appended claims and accompanying drawings where:
FIG. 1 illustrates a side partial cross-sectional view of an insertion cannula and a surgical device in accordance with one embodiment of the present disclosure;
FIG. 2 illustrates a side breakaway, partial cross sectional view of a surgical device placed within a surgical pathway defined by the introducer needle or insertion cannula as shown in FIG. 1;
FIG. 3A illustrates a perspective view of one embodiment according to the invention of a sealing member for disposal at a proximal end of the insertion cannula. The sealing member includes a flared end to prevent entrance of liquid into the space within the surgical pathway;
FIG. 3B illustrates a top view of the sealing member for disposal at a proximal end of an insertion cannula as shown in FIG. 3A, the sealing member having a pilot hole for insertion of a surgical device in a contracted configuration;
FIG. 3C illustrates a top view of the sealing member for disposal at a proximal end of an insertion cannula as shown in FIG. 3A, the sealing member having a pilot hole for insertion of a surgical device in an expanded configuration;
FIG. 4A illustrates a perspective view of one embodiment of a sealing member for disposal at a proximal end of an insertion cannula. The sealing member includes a covering which prevents entrance of liquid into the space within the surgical pathway;
FIG. 4B illustrates a top view of the sealing member for disposal at a proximal end of an insertion cannula as shown in FIG. 4A, the scaling member having a pilot hole for insertion of a surgical device when the pilot hole is in a contracted configuration. As shown in FIG. 4B, the pilot hole is a pin hole;
FIG. 4C illustrates a top view of the sealing member for disposal at a proximal end of an insertion cannula as shown in FIG. 4A, the sealing member having a pilot hole for insertion of a surgical device when the pilot hole is in a contracted configuration. As shown in FIG. 4C, the pilot hole is a slit; and
FIG. 4D illustrates a top view of the sealing member for disposal at a proximal end of an insertion cannula as shown in FIG. 4A, the sealing member having a pilot hole for insertion of a surgical device when the pilot hole is in an expanded configuration.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

Devices for efficient destruction and/or removal of material or substances such as nerve and soft tissue suitable for use in open surgical and/or minimally invasive procedures for the treatment of pain are disclosed. The following description is presented to enable any person skilled in the art to make and use the present disclosure. Descriptions of specific embodiments and applications are provided only as examples and various modifications will be readily apparent to those skilled in the art.

The present disclosure may be understood more readily by reference to the following detailed description of the disclosure presented in connection with the accompanying drawings, which together form a part of this disclosure. It is to be understood that this disclosure is not limited to the specific devices, conditions or parameters described and/or shown herein, and that the terminology used herein is for the purpose of describing particular embodiments by way of example only and is not intended to be limiting of the claimed disclosure.

### Definitions

As used in the specification and including the appended claims, the singular forms "a," "an," and "the" include the plural, and reference to a particular numerical value includes at least that particular value, unless the context clearly dictates otherwise.

Ranges may be expressed herein as from "about" or "approximately" one particular value and/or to "about" or "approximately" another particular value. When such a range is expressed, another embodiment includes from the one particular value and/or to the other particular value.

Similarly, when values are expressed as approximations, by use of the descriptor "about," it will be understood that the particular value forms another embodiment. It is also understood that all spatial references, such as, for example, horizontal, vertical, top, upper, lower, bottom, left and right, are for illustrative purposes only and can be varied within the scope of the disclosure.

For purposes of the description contained herein, with respect to components and movement of components described herein, "forward" or "distal" (and forms thereof) means forward, toward or in the direction of the forward, distal end of the probe portion of the device that is described herein, and "rearward" or "proximal" (and forms thereof) means rearward or away from the direction of the forward, distal end of the probe portion of the device that is described herein. However, it should be understood that these uses of these terms are for purposes of reference and orientation with respect to the description and drawings herein, and are not intended to limit the scope of the claims.

Spatially relative terms such as "under," "below," "lower," "over," "upper," and the like, are used for ease of description to explain the positioning of one element relative to a second element. These terms are intended to encompass different orientations of the device in addition to different orientations than those depicted in the figures. Further, terms such as "first," "second," and the like, are also used to describe various elements, regions, sections, etc. and are also not intended to be limiting. Like terms refer to like elements throughout the description.

As used herein, the terms "having," "containing," "including," "comprising" and the like are open ended terms that indicate the presence of stated elements or features, but do not preclude additional elements or features.

The headings below are not meant to limit the disclosure in any way; embodiments under any one heading may be used in conjunction with embodiments under any other heading.

Reference will now be made in detail to certain embodiments, examples of which are illustrated in the accompanying drawings. While the application will be described in conjunction with the illustrated embodiments, it will be understood that they are not intended to limit the application to those embodiments. On the contrary, the application is intended to cover all alternatives, modifications, and equivalents that may be included within the application as defined by the appended claims.

### Insertion Cannula

The present disclosure provides devices and exemplary methods of use not forming part of the invention for an insertion cannula to insulate a cryoablation probe or other surgical devices. The insertion cannula is inserted into a surgical area on a patient to create a surgical pathway to tissue below the surface of the skin. The insertion cannula provides insulation for the cryoablation probe or other surgical tools which are introduced into the surgical pathway by providing an air gap between the cryoablation probe and the insertion cannula.

In some embodiments, the insertion cannula includes an elastic sealing member on the distal end of the insertion cannula to prevent fluid from entering the surgical pathway. In some embodiments, the sealing member provides a water tight seal that prevents fluid from entering the insertion cannula upon insertion into the patient. In some embodiments, the sealing member comprises a pilot hole for inserting a surgical tool, such as, for example, a needle with an anesthetic drug for the procedure, a RF stimulator for locating the target nerve, or a cryoablation probe. In some embodiments, the elastic sealing member flexes to allow insertion of the surgical tool but maintains a water tight seal around the cylindrical tool or probe. In some embodiments, the sealing member keeps fluid out of the needle. In some embodiments, the sealing member removes any fluid adhered to the side of the surgical tool or cryoablation probe when inserted. In some embodiments, the sealing member keeps the tool or cryoablation probe centered inside the introducer needle or insertion cannula to ensure a uniform air gap, which can be circumferentially disposed about all or a portion of the ablation probe that is within the insertion cannula.

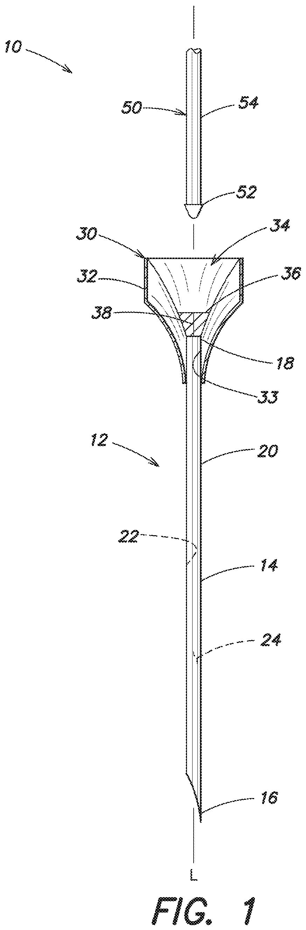

The present disclosure includes an insertion cannula configured to provide insulation to a surgical tool, such as, for example, a cryoablation probe, without damaging and/or destroying adjacent tissues. As illustrated in FIGS. 1-2, the present surgical system 10 comprises an insertion cannula 12 and a surgical tool, such as, for example, cryoablation probe 50. Insertion cannula 12 is configured to receive cryoablation probe 50 and provide insulation to prevent unwanted damage to adjacent tissue. The dimensions of insertion cannula 12, among other things, will depend on the site that needs ablation. For example, the width of the cervical facet is only about 0.5-1.0 cm and about 1.0-2.0 cm for the lumbar facet region. However, it is contemplated that insertion cannula 12 can be used at other surgical locations, and may be sized according to the requirements of the specific surgical location. Thus, the device, in various embodiments, can be designed for these specific areas.

In various embodiments, insertion cannula 12 includes a tube 14 and a sealing member 30 disposed at the insertion cannula's proximal end. Tube 14 extends along a longitudinal axis L and includes an internal passage 24. Tube 14 extends longitudinally between a distal tip 16 and a proximal end 18. A body of tube 14 includes an exterior surface 20 and an inner surface 22. In various embodiments, the cross-sectional shape of tube 14 is circular to accommodate cylindrically shaped surgical tools. However, in other embodiments, tube 14 may be variously configured and the cross-sectional shape of tube 14 may be elliptical, polygonal or irregular.

Suitable materials that tube 14 can be made from, for example, are polyurethane, polyurea, polyether(amide), PEBA, thermoplastic elastomeric olefin, copolyester, and styrenic thermoplastic elastomer, steel, aluminum, stainless steel, titanium, nitinol, tungsten, molybdenum, metal alloys with high non-ferrous metal content and a low relative proportion of iron, carbon fiber, glass fiber, plastics, ceramics or a combination thereof.

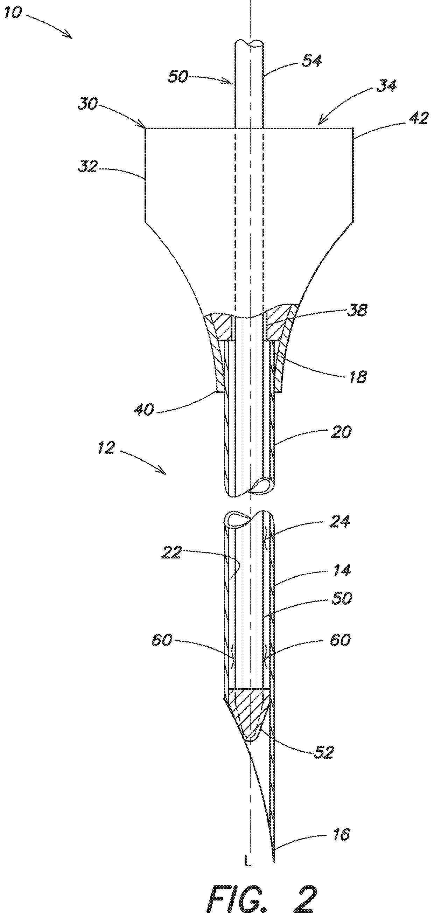

Tip 16 is positioned at the distal end of tube 14 and is configured to penetrate tissue. In some embodiments, as shown in FIGS. 1 and 2, tip 16 is sharp to facilitate penetration through tissue. In some embodiments, tip 16 is blunt to minimize damage done to tissue. In some embodiments, tip 16 is sealed to prevent fluid from entering internal passage 24. In some embodiments, tip 16 includes an opening to allow passage of a therapeutic agent to a predetermined location in a patient's body during a surgical procedure. The opening may have a regular or irregular shape including arcuate, round, square, oblong, kidney shaped, crescent, or beveled shaped. In some embodiments, therapeutic agents can be delivered to the surgical site via the openings.

Internal passage 24 creates a surgical pathway configured to receive a longitudinal surgical tool, such as, for example, probe 50. In some embodiments, internal passage 24 includes a diameter sized to be wider than a diameter of probe 50. In some embodiments, the diameter of internal passage 24 is sized to leave space between inner surface 22 of tube 14 and an outer surface 54 of probe 50, as discussed herein. The space between tube 14 and probe 50 creates an air gap 60 for insulation. In various embodiments, the cross-sectional shapes of tube 14 and probe 50 are circular and the diameter of inner surface 22 of tube 14 is sufficiently larger than the diameter of outer surface 54 of probe 50 to insulate probe 50 and prevent unwanted tissue damage during surgery. Air gap 60 uniformly surrounds a portion or the entire outer surface 54 within internal passage 24 to facilitate insulation of probe 50. In some embodiments, air gap 60 includes a uniform radial thickness to surround probe 50. In such embodiments, tube 14 may be cylindrically shaped to fit over a cylindrically shaped portion of probe 50. However, in some embodiments, air gap 60 may be irregular. That is, in some embodiments, air gap 60 may be thicker in certain dimensions than other dimensions, and certain portions of outer surface 54 may be more insulated than other portions.

The air gap allows the appropriate insulation and allows specific and controlled ice ball formation about the probe and/or insertion cannula so that desired soft tissue and/or nerve tissue is ablated, while reducing or inhibiting unwanted collateral tissue damage.

In various embodiments, tube 14 is configured to provide a barrier to prevent fluid from the body of a patient from entering internal passage 24. In some embodiments, tube 14 is free from openings or apertures to maintain a fluid proof barrier to maintain the effectiveness of air gap 60 as an insulator. Sealing member 30 is configured to maintain a fluid proof barrier at proximal end 18 of tube 14. In some embodiments, sealing member 30 is integrally formed with tube 14, such that sealing member 30 is not removable therefrom. In some embodiments, sealing member 30 is separately formed from tube 14 and is attachable to proximal end 18. A body 32 of sealing member 30 may include an engagement surface 33 configured to engage proximal end 18 of tube 14. In one embodiment, engagement surface 33 is rubberized to provide a fluid proof friction fit over proximal end 18. In one embodiment, engagement surface 33 may include an adhesive material to engage proximal end 18. The adhesive material may include viscous, semi-solid, plastic materials, asphalts, natural and synthetic resins, or the like. In other embodiments, sealing member 30 may engage proximal end 18 through use of threads, latches, snaps, hooks, or the like.

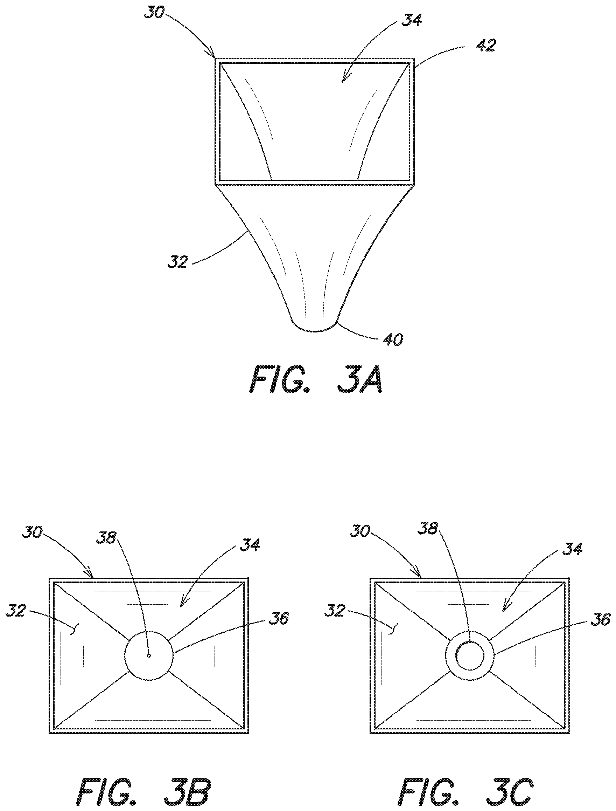

In the embodiment according to the invention, as shown in FIGS. 3A-3C, sealing member 30 includes a flared body 32 extending between a distal end 40 and a proximal end 42. Body 32 includes four flared sides which are wider at proximal end 42 than at distal end 40. The flared shape of body 32 serves a number of purposes. For example, the flared shape allows for easy access to internal passage 24 during a surgical procedure. Additionally, in some applications, the flared shape may act as an anchor at the surface of the skin of a patient when creating a surgical pathway to deep tissue during a surgical procedure. The flared shape of body 32 also maintains a barrier against surrounding fluid while also allowing proximal end 18 of tube 14 to submerge below a fluid level.

Body 32 defines four flared sides such that the cross-sectional shape of sealing member 30 toward proximal end 42 is a quadrilateral. The flared shape of body 32 defines an interior cavity 34. Cavity 34 extends distally from proximal end 42 and terminates at a fluid proof surface 36. Cavity 34 narrows toward surface 36 to aid in the placement and advancement of a surgical tool through internal passage 24. Surface 36 is configured to abut tube 14 at proximal end 18 and to prevent fluid from internal passage 24. Surface 36 comprises an elastic material and is deformable to allow passage of probe 50 through surface 36 into internal passage 24.

Surface 36 comprises a central pilot hole 38. As shown in FIG. 3B, pilot hole 38 is biased to the rest position. In some embodiments, as shown for example in FIG. 3B, pilot hole 38 is a pin hole which is too small to allow passage of fluid while in the rest position. Pilot hole 38 is elastic and movable between a rest position, such as a contracted configuration or first configuration, and a non-rest position, such as an expanded configuration. When in the contracted configuration or first configuration as shown in FIG. 3B, pilot hole 38 is impenetrable to fluids (e.g., gas, liquid, etc.) to maintain a fluid proof seal over internal passage 24. When in the expanded configuration or second configuration as shown in FIG. 3C, pilot hole 38 is wide enough to allow only probe 50 through, while also deflecting residual fluids on outer surface 54 of probe 50.

Pilot hole extends through a depth of body 32 to create a channel when in the expanded configuration. Pilot hole 38 is sized to be deep enough to stabilize probe 50 upon insertion into internal passage 24 such that probe 50 is centered relative to inner surface 22 of tube 14.

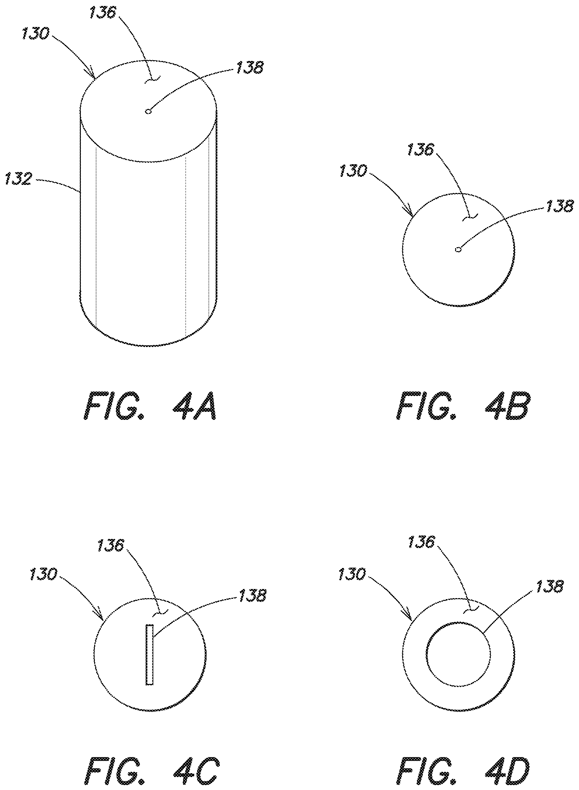

In some embodiments, as shown in FIGS. 4A-4D, insertion cannula 12 includes a sealing member 130, similar to sealing member 30. Sealing member 130 is configured to maintain a fluid proof barrier at proximal end 18 of tube 14. In some embodiments, sealing member 130 is integrally formed with tube 14, such that sealing member 130 is not removable therefrom. In some embodiments, sealing member 130 is separately formed from tube 14 and is attachable to proximal end 18. A body 132 of sealing member 130 may include an engagement surface configured to engage proximal end 18 of tube 14. Sealing member 130 includes a fluid proof surface 136 that abuts tube 14 at proximal end 18 and to prevent fluid from entering internal passage 24. Surface 136 comprises an elastic material and is deformable to allow passage of probe 50 through surface 136 into internal passage 24.

In some embodiments, sealing member 130 comprises elastic material and has a modulus of elasticity in the range of about 1x10² to about 6x10⁵ dyn/cm², or 2x10⁴ to about 5x10⁵ dyn/cm², or 5x10⁴ to about 5x10⁵ dyn/cm².

In some embodiments, surface 136 comprises a central pilot hole 138. In one embodiment, as shown in FIGS. 4A-4B, pilot hole 138 comprises a pin hole. In one embodiment, as shown in FIG. 4C, pilot hole 138 comprises a central slit. Pilot hole 138 is biased to the rest position. In some embodiments, pilot hole 138 is too small to allow passage of fluid while in the rest position. Pilot hole 138 is elastic and movable between a rest position, such as a contracted configuration, and a non-rest position, such as an expanded configuration. When in the contracted configuration as shown in FIGS. 4B and 4C, pilot hole 138 is impenetrable to fluids to maintain a fluid proof seal over internal passage 24. When in the expanded configuration as shown in FIG. 4D, pilot hole 138 is wide enough to allow only probe 50 through, while also deflecting residual fluids on outer surface 54 of probe 50.

In various embodiments, the tip of surgical system 10 comprises a telescopic configuration. The tips can be manually or electronically movable so as to place the tips into a particular position within a surgical site. In certain embodiments, all or some of the tips comprise a telescopic configuration. In some embodiments, the tip is a navigational tool used to guide surgical system 10 into a surgical site.

In some embodiments, the tips of surgical system 10 each comprise indicia, for example a depth indicator that may include an analog, such as, for example, a dial with a numerical indicator of angle and/or digital display, such as, for example, LED and/or LCD. The graduations may represent various indicia, such as, for example, numerical, alphabetic and/or specific conditions/orientations, such as initial depth and/or final depth of penetration into the nerve and/or tissue.

In various embodiments, at a proximal end, insertion cannula 12 can be operatively connected to a vacuum (not shown) for providing suction to ablated nerve and/or tissue. The vacuum may be used to transmit vacuum from a vacuum source (not shown) to a receiving aperture (not shown) connected to insertion cannula 12. Any suitable aspirator, cylindrical or otherwise, or other mechanism that creates vacuum upon the movement of an actuating member thereof, may be utilized as a vacuum source. The vacuum can be in communication with the tips of insertion cannula 12 for providing suction to remove ablated nerve and/or soft tissue.

### Cryoablation

Cryoablation devices have been available to surgeons to treat many medical conditions, for example, in the treatment of tumors in lung, liver, kidney and other body organs. Cryoablation has also been used for treatment of tumors, cardiac arrhythmias, chronic and post-operative pain, bone fracture and soft tissue wounds.

Cold temperatures have been used to decrease inflammation and to relieve pain since the ancient Egyptians. Liquid air and carbon dioxide were used to treat skin lesions in the beginning of the twentieth century. In 1950, liquid nitrogen was introduced into clinical practice for the cryosurgical ablation of a variety of skin diseases and allowed for deeper tissue to be treated with cryoablation. In 1961, a liquid nitrogen probe was developed and was used to treat Parkinson's disease as well as inoperable brain tumors. From 1980-2000, systems emerged based on an advanced gas expansion method known as the Joule-Thomson Principle. This principle allows for temperature change of a gas or liquid when it is forced through a valve or porous sealing member while being kept insulated so that no heat is exchanged with the environment. The refrigerant could be stored at room temperature and the difficulties associated with supplying liquid nitrogen to the operating room disappeared. Three main refrigerants were utilized: nitric oxide, liquid nitrogen and argon. For over 20 years, rigid cryoprobes have existed for percutaneous use or in open invasive surgical procedures. For example, cryoprobes are used for freezing a range of lesions from prostate tissue to metastatic cancers of the liver. Neuronal tissue has been frozen with such devices for the relief of pain.

Current cryoablation procedures and technique employ cryoprobes that utilize single needles that form an ice ball centered around a tip that are disposed at a surgical site. Because current cryoablation probes are not insulated, there is an increase in heat loss for adjacent tissues at a surgical site. The heat loss leads to tissue freezing and potential necrosis. In addition, there is an increase in radiation exposure to the surgical site. Therefore, the probe of the present disclosure decreases the amount of tissue damage and/or destruction, as well as contamination that potentially can occur for enhanced ablation.

The present disclosure includes a cryoablation probe capable of efficiently ablating multiple areas of a surgical site without damaging and/or destroying adjacent tissues. As illustrated in FIGS. 1-2, the present ablation surgical system 10 comprises a probe 50 having a cylindrical outer surface 54. In various embodiments, surgical system 10 includes multiple probes. The dimensions of the device, among other things, will depend on the site that needs ablation. In various embodiments, the diameter of probe 50 is less than the diameter of inner surface 22 of tube 14 such that a space exists between inner surface 22 and outer surface 54 of probe 50.

Some examples of lengths of probe 50, may include, but are not limited to, from about 50 to 150 mm in length, for example, about 50 mm for cervical facet use, about 100 mm for lumbar facet use for a standard adult and about 150 mm for an obese adult patient. The thickness of probe 50 will depend on the site that needs ablation and/or the particular embodiment of the device. The thickness of probe 50 is about 20 gauge. In some embodiments, probe 50 can be about 17 to about 22 gauge. In various embodiments, the thickness includes, but is not limited to, from about 0.05 to about 1.655 mm In some embodiments, probe 50 can be increasing and or decreasing in thickness throughout probe 50. In some embodiments, probe 50 may be tapered and/or angled. Probe 50 may be the widest or smallest acceptable diameter or a diameter in between for insertion into a human or animal body. In some embodiments, the widest diameter is typically about 14 gauge, while the smallest diameter is about 26 gauge.

Suitable materials that probe 50 can be made from, for example, are polyurethane, polyurea, polyether(amide), PEBA, thermoplastic elastomeric olefin, copolyester, and styrenic thermoplastic elastomer, steel, aluminum, stainless steel, titanium, nitinol, tungsten, molybdenum, metal alloys with high non-ferrous metal content and a low relative proportion of iron, carbon fiber, glass fiber, plastics, ceramics or a combination thereof.

In some embodiments, probe 50 comprises an elastic member 52 sized to contact the internal passage of the insertion cannula. Elastic member 52 may be attached to the tip of probe 50 or to a distal portion near the tip. Elastic member 52 is attached to a distal end of outer surface 54 and is sized to tightly fit within internal passage 24 of tube 14. Elastic member 52 includes a circular outer surface to contact inner surface 22. In some embodiments, to further ensure that no fluid is present in the surgical pathway of the introducer needle, elastic member 52 includes a slightly larger diameter than probe 50 so that when probe 50 is advanced through internal passage 24, elastic member 52 brushes away any fluids adhered to inner surface 22. In some embodiments, elastic member 52 is placed on the distal tip of probe 50. In some embodiments, elastic member 52 can be mechanically fixed to the metal cryoprobe. In some embodiments, elastic member 52 is removable from probe 50. In various embodiments, elastic member 52 of probe 50 is configured for threaded engagement with a distal end of probe 50. Elastic member 52 may be disposed within probe 50 via friction fit, lock and key, male/female, clip, keyway, and/or bracket engagement. In some embodiments, elastic member 52 is approximately 1-2cm in length and has a wider diameter than probe 50. In some embodiments, when probe 50 is inserted within the tube 14, elastic member 52 pushes any fluid drops that may have entered out of an opening at distal tip 16 of tube 14.

In various embodiments, engagement of elastic member 52 with inner surface 22 produces an air gap 60 at outer surface 54 of probe 50 and surface 22 of tube 14. Air gap 60 forms an air seal that creates insulation around probe 50 to diminish and/or prevent collateral tissue damage at a surgical site. Air gap 60 uniformly surrounds the entirety of outer surface 54 within internal passage 24 to facilitate insulation of probe 50. In some embodiments, air gap 60 includes a uniform radial thickness to surround probe 50. In such embodiments, tube 14 may be cylindrically shaped to fit over a cylindrically shaped portion of probe 50. However, in some embodiments, air gap 60 may be irregular. That is, in some embodiments, air gap 60 may be thicker in certain dimensions than other dimensions, and certain portions of outer surface 54 may be more insulated than other portions.

In various embodiments, probe 50 comprises an insulated material such as, for example, a heat resistant plastic. Examples of such heat resistant plastics include, but are not limited to PMMA, PET, PEEK, PLA, PLGA, PVC and/or HDPE. In various embodiments, tube 14 comprises other insulated materials including, but not limited to glass, ceramic, porcelain, composite polymers and/or rubber. Examples of such rubbers include, but are not limited to silicone, flurosilicone, isobutylene-isoprene copolymer, chlorobutyl, fluroelastomers, and/or polychloroprene.

The temperature for cryoablation of the device can be selected by the user and can vary as needed. For example, the temperature that can be selected can be from - 180° C, -170° C, -160° C, -150° C, -140° C, -130° C, -120° C, -110° C, -100° C, -50° C, -40° C, -30° C, -20° C, -10° C, -5° C or to about 0° C or any temperature in between these numbers. The device comprises air gap 60 between at least portions or all of the inner surface 22 of tube 14 and outer surface 54 of probe 50 to provide insulation from these temperatures.

After a period of time, an ice ball forms and begins to ablate when a portion of the tip of probe 50 is adjacent to nerve and/or soft tissue and when the temperature of the tip of probe 50 decreases from about -40° C to about -160° C. The temperature at the surface of the ice ball is 0° C. The temperature declines exponentially towards a cool center where it reaches about -170° C. The sphere creates a zone of complete ablation (approximately -20° C) typically located within the ice ball at approximately half way between the center of the ball and its outer surface. In various embodiments, nerve and/or soft tissue is completely ablated in about 3 to about 16 minutes. In various embodiments, nerve and/or soft tissue is completely ablated in about 3 to about 9 minutes. In some embodiments, the ice ball is not a complete ice ball; for example, a partial or half an ice ball can be formed for complete ablation.

In various embodiments, the device or tip of the device is coated with an antimicrobial coating and/or agents. The antimicrobial coating can include, for example, antibiotics, antifungal, antiviral agents or the like. Antimicrobial agents to treat infection include, by way of example and not limitation, antiseptic agents, antibacterial agents; quinolones and in particular fluoroquinolones (e.g., norfloxacin, ciprofloxacin, lomefloxacin, ofloxacin, etc.), aminoglycosides (e.g., gentamicin, tobramycin, etc.), glycopeptides (e.g., vancomycin, etc.), lincosamides (e.g., clindamycin), cephalosporins (e.g., first, second, third generation) and related beta-lactams, macrolides (e.g., azithromycin, erythromycin, etc.), nitroimidazoles (e.g., metronidazole), penicillins, polymyxins, tetracyclines, or combinations thereof.

Some exemplary antimicrobial agents include, by way of illustration and not limitation, acedapsone; acetosulfone sodium; alamecin; alexidine; amdinocillin; amdinocillin pivoxil; amicycline; amifloxacin; amifloxacin mesylate; amikacin; amikacin sulfate; aminosalicylic acid; aminosalicylate sodium; amoxicillin; amphomycin; ampicillin; ampicillin sodium; apalcillin sodium; apramycin; aspartocin; astromicin sulfate; avilamycin; avoparcin; azithromycin; azlocillin; azlocillin sodium; bacampicillin hydrochloride; bacitracin; bacitracin methylene disalicylate; bacitracin zinc; bambermycins; benzoylpas calcium; berythromycin; betamicin sulfate; biapenem; biniramycin; biphenamine hydrochloride; bispyrithione magsulfex; butikacin; butirosin sulfate; capreomycin sulfate; carbadox; carbenicillin disodium; carbenicillin indanyl sodium; carbenicillin phenyl sodium; carbenicillin potassium; carumonam sodium; cefaclor; cefadroxil; cefamandole; cefamandole nafate; cefamandole sodium; cefaparole; cefatrizine; cefazaflur sodium; cefazolin; cefazolin sodium; cefbuperazone; cefdinir; cefepime; cefepime hydrochloride; cefetecol; cefixime; cefmenoxime hydrochloride; cefmetazole; cefmetazole sodium; cefonicid monosodium; cefonicid sodium; cefoperazone sodium; ceforanide; cefotaxime sodium; cefotetan; cefotetan disodium; cefotiam hydrochloride; cefoxitin; cefoxitin sodium; cefpimizole; cefpimizole sodium; cefpiramide; cefpiramide sodium; cefpirome sulfate; cefpodoxime proxetil; cefprozil; cefroxadine; cefsulodin sodium; ceftazidime; ceftibuten; ceftizoxime sodium; ceftriaxone sodium; cefuroxime; cefuroxime axetil; cefuroxime pivoxetil; cefuroxime sodium; cephacetrile sodium; cephalexin; cephalexin hydrochloride; cephaloglycin; cephaloridine; cephalothin sodium; cephapirin sodium; cephradine; cetocycline hydrochloride; cetophenicol; chloramphenicol; chloramphenicol palmitate; chloramphenicol pantothenate complex; chloramphenicol sodium succinate; chlorhexidine phosphanilate; chloroxylenol; chlortetracycline bisulfate; chlortetracycline hydrochloride; cinoxacin; ciprofloxacin; ciprofloxacin hydrochloride; cirolemycin; clarithromycin; clinafloxacin hydrochloride; clindamycin; clindamycin hydrochloride; clindamycin palmitate hydrochloride; clindamycin phosphate; clofazimine; cloxacillin benzathine; cloxacillin sodium; chlorhexidine, cloxyquin; colistimethate sodium; colistin sulfate; coumermycin; coumermycin sodium; cyclacillin; cycloserine; dalfopristin; dapsone; daptomycin; demeclocycline; demeclocycline hydrochloride; demecycline; denofungin; diaveridine; dicloxacillin; dicloxacillin sodium; dihydrostreptomycin sulfate; dipyrithione; dirithromycin; doxycycline; doxycycline calcium; doxycycline fosfatex; doxycycline hyclate; droxacin sodium; enoxacin; epicillin; epitetracycline hydrochloride; erythromycin; erythromycin acistrate; erythromycin estolate; erythromycin ethylsuccinate; erythromycin gluceptate; erythromycin lactobionate; erythromycin propionate; erythromycin stearate; ethambutol hydrochloride; ethionamide; fleroxacin; floxacillin; fludalanine; flumequine; fosfomycin; fosfomycin tromethamine; fumoxicillin; furazolium chloride; furazolium tartrate; fusidate sodium; fusidic acid; ganciclovir and ganciclovir sodium; gentamicin sulfate; gloximonam; gramicidin; haloprogin; hetacillin; hetacillin potassium; hexedine; ibafloxacin; imipenem; isoconazole; isepamicin; isoniazid; josamycin; kanamycin sulfate; kitasamycin; levofuraltadone; levopropylcillin potassium; lexithromycin; lincomycin; lincomycin hydrochloride; lomefloxacin; lomefloxacin hydrochloride; lomefloxacin mesylate; loracarbef; mafenide; meclocycline; meclocycline sulfosalicylate; megalomicin potassium phosphate; mequidox; meropenem; methacycline; methacycline hydrochloride; methenamine; methenamine hippurate; methenamine mandelate; methicillin sodium; metioprim; metronidazole hydrochloride; metronidazole phosphate; mezlocillin; mezlocillin sodium; minocycline; minocycline hydrochloride; mirincamycin hydrochloride; monensin; monensin sodiumr; nafcillin sodium; nalidixate sodium; nalidixic acid; natainycin; nebramycin; neomycin palmitate; neomycin sulfate; neomycin undecylenate; netilmicin sulfate; neutramycin; nifuiradene; nifuraldezone; nifuratel; nifuratrone; nifurdazil; nifurimide; nifiupirinol; nifurquinazol; nifurthiazole; nitrocycline; nitrofurantoin; nitromide; norfloxacin; novobiocin sodium; ofloxacin; onnetoprim; oxacillin and oxacillin sodium; oximonam; oximonam sodium; oxolinic acid; oxytetracycline; oxytetracycline calcium; oxytetracycline hydrochloride; paldimycin; parachlorophenol; paulomycin; pefloxacin; pefloxacin mesylate; penamecillin; penicillins such as penicillin g benzathine, penicillin g potassium, penicillin g procaine, penicillin g sodium, penicillin v, penicillin v benzathine, penicillin v hydrabamine, and penicillin v potassium; pentizidone sodium; phenyl aminosalicylate; piperacillin sodium; pirbenicillin sodium; piridicillin sodium; pirlimycin hydrochloride; pivampicillin hydrochloride; pivampicillin pamoate; pivampicillin probenate; polymyxin b sulfate; porfiromycin; propikacin; pyrazinamide; pyrithione zinc; quindecamine acetate; quinupristin; racephenicol; ramoplanin; ranimycin; relomycin; repromicin; rifabutin; rifametane; rifamexil; rifamide; rifampin; rifapentine; rifaximin; rolitetracycline; rolitetracycline nitrate; rosaramicin; rosaramicin butyrate; rosaramicin propionate; rosaramicin sodium phosphate; rosaramicin stearate; rosoxacin; roxarsone; roxithromycin; sancycline; sanfetrinem sodium; sarmoxicillin; sarpicillin; scopafungin; sisomicin; sisomicin sulfate; sparfloxacin; spectinomycin hydrochloride; spiramycin; stallimycin hydrochloride; steffimycin; streptomycin sulfate; streptonicozid; sulfabenz; sulfabenzamide; sulfacetamide; sulfacetamide sodium; sulfacytine; sulfadiazine; sulfadiazine sodium; sulfadoxine; sulfalene; sulfamerazine; sulfameter; sulfamethazine; sulfamethizole; sulfamethoxazole; sulfamonomethoxine; sulfamoxole; sulfanilate zinc; sulfanitran; sulfasalazine; sulfasomizole; sulfathiazole; sulfazamet; sulfisoxazole; sulfisoxazole acetyl; sulfisboxazole diolamine; sulfomyxin; sulopenem; sultamricillin; suncillin sodium; talampicillin hydrochloride; teicoplanin; temafloxacin hydrochloride; temocillin; tetracycline; tetracycline hydrochloride; tetracycline phosphate complex; tetroxoprim; thiamphenicol; thiphencillin potassium; ticarcillin cresyl sodium; ticarcillin disodium; ticarcillin monosodium; ticlatone; tiodonium chloride; tobramycin; tobramycin sulfate; tosufloxacin; trimethoprim; trimethoprim sulfate; trisulfapyrimidines; troleandomycin; trospectomycin sulfate; tyrothricin; vancomycin; vancomycin hydrochloride; virginiamycin; zorbamycin; or combinations thereof.

In some embodiments, the device can be coated with an antiviral agent. Antiviral agents can include, but are not limited to, vidarabine, acyclovir, famciclovir, valacyclovir, gancyclovir, valganciclovir, nucleoside-analog reverse transcriptase inhibitors (such as AZT (zidovudine), ddl (didanosine), ddC (zalcitabine), d4T (stavudine), and 3TC (lamivudine)), nevirapine, delavirdine, protease inhibitors (such as, saquinavir, ritonavir, indinavir, and nelfinavir), ribavirin, amantadine, rimantadine, neuraminidase inhibitors (such as zanamivir and oseltamivir), pleconaril, cidofovir, foscarnet, and/or interferons.

Depending on the particular embodiment, the size of the probe tip determines the size of the ice ball formed. In some embodiments, the length of the tip is about 0.5 to about 2 mm for smaller ice balls and from about 3 to about 6 mm for larger ice balls.

In certain embodiments, probe 50 may include switches for manually controlling the operation of probe 50 by a medical practitioner. The switches can provide functions such as powering on/off, cooling, and predetermined cycles of heating and cooling by selectively and controllably communicating probe 50 with an external material container.

In some embodiments, different monitors of temperature, gas pressure and location on surgical system 10 can be attached to surgical system 10. In some embodiments, thermal sensors may be used for measuring the temperature of the material and/or the tips. In some embodiments, surgical system 10 can be operatively connected to semi-steerable or navigational sources for easier guidance into tissues. In various embodiments, the navigational sources can be coupled with a pre-procedure such as for example, CT, MRI, PET scan, etc. so that the target nerve or soft tissue to be ablated can be identified and accurately located during the procedure.

In various embodiments, the device may include radiographic markers to help indicate position on imaging procedures (e.g., CT scan, X-ray, fluoroscopy, PET scan, etc.). These may be disposed on or a portion of the device and include, but are not limited to, barium, calcium phosphate, and/or metal beads. Exemplary methods for ablation not forming part of the invention

The present disclosure also provides exemplary methods for destroying or removing nerve and/or soft tissue. In some examples, the methods comprise disposing an insertion cannula at a surgical site, the insertion cannula comprising a tube and a sealing member, the tube having an exterior surface comprising a distal tip configured to penetrate tissue and an interior surface that defines an internal passage, the tube having a longitudinal axis and the interior passage extending along the longitudinal axis, wherein the sealing member is disposed with a proximal end of the tube; and placing a surgical tool through the internal passage of the insertion cannula to the nerve and/or soft tissue to ablate the nerve and/or soft tissue.

In some examples, the site is a facet joint or a plurality of facet joints and the tip of the probe is placed adjacent to nerve and/or soft tissue to form an asymmetrical ice ball configured for ablating the nerve and/or the soft tissue. In various examples, an ice ball forms in about 2 to about 8 minutes and the nerve is ablated from about 3 to about 16 minutes.

In several examples, the methods disclosed herein include operatively coupling the device to a source of navigational capability to allow easier pushing through the tissues. In various examples, the methods of ablation disclosed herein can include a pre-procedure step wherein the device can be coupled to a CT or MRI machine so that the target nerve and/or soft tissue to be ablated can be identified and accurately located during the ablation procedure.

The methods for ablation described hereinabove allow complete destruction of the nerve avoiding the problems and partial effectiveness of current cryoablation devices available in the art, and also allow for more complete removal of soft tissue that is causing stenosis pain symptoms.

In various embodiments, kits are provided that include surgical system 10. The kits can include at least one insertion cannula 12 and at least one probe 50. In some embodiments, probe 50 and/or the tip is made reusable for multiple procedures after cleaning and sterilization.

Specific clinical application of this instrument include destruction of nerves causing facet and discogenic back and leg pain, destruction of soft tissue causing stenosis pain symptoms, and many other orthopedic and oral maxillofacial pain. Many other painful conditions associated with arthroscopic, otolaryngological or spinal procedures could use the ablation devices and methods of using these ablation devices described herein.

It will be apparent to those skilled in the art that various modifications and variations can be made to various embodiments described herein without departing from the scope of the teachings herein. Thus, it is intended that various embodiments cover other modifications and variations of various embodiments within the scope of the appended claims.

## Claims

1. A surgical device comprising an insertion cannula (12) which comprises a tube (14) having a proximal end (18), an exterior surface (20) comprising a distal tip (16) configured to penetrate tissue, and an interior surface (22) that defines an internal passage (24) of the insertion cannula (12), the internal passage (24) extending along a longitudinal axis (L) of the insertion cannula (12) and configured to receive a surgical tool, a sealing member (30) contacting the proximal end (18) of the tube (14) and configured to provide a fluid seal to prevent fluid from entering or leaving the proximal end (18) of the tube (14); wherein
the sealing member (30) includes a flared body (32) extending between a distal end (40) and a proximal end (42); wherein the flared body (32) defines four flared sides such that the cross-scctional shape of scaling member (30) toward the proximal end (42) is a quadrilateral, wherein the four flared sides are wider at the proximal end (42) than at the distal end (40);
wherein the flared shape of body (32) defines an interior cavity (34), wherein the cavity (34) extends distally from the proximal end (42) and terminates at a fluid proof surface (36), wherein the cavity (34) narrows toward the fluid proof surface (36); wherein
the fluid proof surface (36) is configured to abut the tube (14) at the proximal end (18) and to prevent fluid from the internal passage (24); and wherein
the fluid proof surface (36) comprises an elastic material and is deformable to allow passage of the surgical tool through the surface (36) into the internal passage (24).

2. A surgical device of claim 1, wherein the surgical tool is an ablation probe (50), and wherein the insertion cannula (12) comprises an opening at a distal end configured to allow the ablation probe (50) to extend through the opening to ablate tissue adjacent the opening.

3. A surgical device of claim 1, wherein the surgical tool is an ablation probe (50), and wherein the sealing member (30) is aligned with the internal passage (24), and an outer surface (54) of the ablation probe (50) has a smaller diameter than a diameter of the interior surface (22) of the insertion cannula (12) such that when a portion of the ablation probe (50) is within the insertion cannula (12), an air gap (60) is provided in the internal passage (24) circumferentially about the ablation probe (50).

4. A surgical device of claim 1, wherein the surgical tool is an ablation probe (50), and wherein the fluid proof surface (36) comprises a pilot hole (38) configured to receive the ablation probe (50).

5. A surgical device of claim 4, wherein the sealing member (30) is elastic and the pilot hole (38) is movable between a first configuration and a second configuration, the first configuration being sized to prevent fluid from entering the insertion cannula (12) and the second configuration being sized to receive the ablation probe (50), wherein, optionally,
(i) the pilot hole (38) comprises a larger diameter when in the second configuration than when in the first configuration, and/or
(ii) the pilot hole (38) is biased in the first configuration to provide an air tight friction fit for the ablation probe (50) being inserted through the pilot hole (38).

6. A surgical device of claim 4, wherein the pilot hole (38) defines a channel positioned concentrically with the internal passage (24) of the insertion cannula (12), the channel configured to guide the ablation probe (50) through the internal passage (24) such that the insertion cannula (12) and the ablation probe (50) are spaced apart by an air gap (60).

7. A surgical device of claim 1, wherein the surgical tool is an ablation probe (50), and wherein the sealing member (30) is attached to the insertion cannula (12) via a rubberized friction fit.

8. A surgical system (10) for use in a minimally invasive surgical procedure, the system comprising a surgical device according to claim 1 and a surgical tool.

9. A surgical system (10) of claim 8, wherein the surgical tool comprises an elastic member (52) sized to contact the internal passage (24) of the insertion cannula (12).

10. A surgical system (10) of claim 8, wherein the surgical tool comprises a surgical needle, an RF stimulator, or a cryoablation probe.

11. A surgical system (10) of claim 8, wherein the distal tip (16) comprises an opening to allow passage of a therapeutic agent to a surgical site.

12. A surgical system of claim 8, wherein the surgical tool is an ablation probe (50), wherein the fluid proof surface (36) comprises a pilot hole (38) that defines a channel positioned concentrically with the internal passage (24) of the insertion cannula (12), the channel configured to guide the ablation probe (50) through the internal passage (24) such that the insertion cannula (12) and the ablation probe (50) are spaced apart by an air gap (60).

13. A surgical system of claim 12, wherein the sealing member (30) is elastic and the pilot hole (38) is movable between a first configuration and a second configuration, the first configuration being a smaller diameter and sized to prevent fluid from entering the insertion cannula (12) and the second configuration being a larger diameter than the first configuration and sized to receive the ablation probe (50).

14. A surgical system of claim 8, wherein the surgical tool is positioned concentrically within the internal passage (24) of the insertion cannula (12), the internal passage (24) configured to guide the surgical tool through the internal passage (24) such that the insertion cannula (12) and the surgical tool are spaced apart by an air gap (60).

15. A surgical system of claim 8, wherein the sealing member (30) is attached to the insertion cannula (12) via a rubberized friction fit.

16. A surgical system of claim 8, wherein the insertion cannula (12) comprises an opening at a distal end configured to allow the surgical tool to extend through the opening to cut tissue adjacent the opening.

## Patentansprüche

1. Chirurgische Vorrichtung, die eine Einführkanüle (12) umfasst, die einen Schlauch (14) umfasst, der ein proximales Ende (18), eine Außenoberfläche (20), die eine distale Spitze (16) umfasst, die konfiguriert ist, um Gewebe zu durchdringen, und eine Innenoberfläche (22) aufweist, die einen inneren Durchgang (24) der Einführkanüle (12) definiert, wobei sich der innere Durchgang (24) entlang einer Längsachse (L) der Einführkanüle (12) erstreckt und konfiguriert ist, um ein chirurgisches Werkzeug aufzunehmen, wobei ein Abdichtungselement (30) das proximale Ende (18) des Schlauchs (14) berührt und konfiguriert ist, um eine Fluidabdichtung bereitzustellen, um Fluid daran zu hindern, in das proximale Ende (18) des Schlauchs (14) einzutreten oder aus diesem auszutreten; wobei
das Abdichtungselement (30) einen trichterförmig aufgeweiteten Körper (32) beinhaltet, der sich zwischen einem distalen Ende (40) und einem proximalen Ende (42) erstreckt; wobei der trichterförmig aufgeweitete Körper (32) vier trichterförmig aufgeweitete Seiten derart definiert, dass die Querschnittsform des Abdichtungselements (30) in Richtung des proximalen Endes (42) ein Viereck ist, wobei die vier trichterförmig aufgeweiteten Seiten an dem proximalen Ende (42) breiter als an dem distalen Ende (40) sind;
wobei die trichterförmig aufgeweitete Form des Körpers (32) einen Innenhohlraum (34) definiert, wobei sich der Hohlraum (34) aus dem proximalen Ende (42) distal erstreckt und an einer fluiddichten Oberfläche (36) endet, wobei sich der Hohlraum (34) in Richtung der fluiddichten Oberfläche (36) verengt; wobei
die fluiddichte Oberfläche (36) konfiguriert ist, um an den Schlauch (14) an dem proximalen Ende (18) anzustoßen und um Fluid daran zu hindern, in den inneren Durchgang (24) zu gelangen; und wobei
die fluiddichte Oberfläche (36) ein elastisches Material umfasst und verformbar ist, um den Durchgang des chirurgischen Werkzeugs durch die Oberfläche (36) in den inneren Durchgang (24) zu ermöglichen.

2. Chirurgische Vorrichtung nach Anspruch 1, wobei das chirurgische Werkzeug eine Ablationssonde (50) ist und wobei die Einführkanüle (12) eine Öffnung an einem distalen Ende umfasst, die konfiguriert ist, um der Ablationssonde (50) zu ermöglichen, sich durch die Öffnung zu erstrecken, um an Gewebe angrenzend an die Öffnung eine Ablation durchzuführen.

3. Chirurgische Vorrichtung nach Anspruch 1, wobei das chirurgische Werkzeug eine Ablationssonde (50) ist und wobei das Abdichtungselement (30) an dem inneren Durchgang (24) ausgerichtet ist und eine äußere Oberfläche (54) der Ablationssonde (50) einen kleineren Durchmesser als ein Durchmesser der Innenoberfläche (22) der Einführkanüle (12) derart aufweist, dass, wenn sich ein Abschnitt der Ablationssonde (50) innerhalb der Einführkanüle (12) befindet, ein Luftspalt (60) in dem inneren Durchgang (24) um die Ablationssonde (50) herum umlaufend bereitgestellt wird.

4. Chirurgische Vorrichtung nach Anspruch 1, wobei das chirurgische Werkzeug eine Ablationssonde (50) ist und wobei die fluiddichte Oberfläche (36) ein Führungsloch (38) umfasst, das konfiguriert ist, um die Ablationssonde (50) aufzunehmen.

5. Chirurgische Vorrichtung nach Anspruch 4, wobei das Abdichtungselement (30) elastisch ist und das Führungsloch (38) zwischen einer ersten Konfiguration und einer zweiten Konfiguration bewegbar ist, wobei die erste Konfiguration bemessen ist, um Fluid daran zu hindern, in die Einführkanüle (12) einzutreten, und die zweite Konfiguration bemessen ist, um die Ablationssonde (50) aufzunehmen, wobei, optional,
(i) das Führungsloch (38), wenn es sich in der zweiten Konfiguration befindet, einen größeren Durchmesser aufweist als wenn es sich in der ersten Konfiguration befindet, und/oder
(ii) das Führungsloch (38) in der ersten Konfiguration vorgespannt ist, um eine hermetisch verschlossene Reibpassung für die Ablationssonde (50) bereitzustellen, die durch das Führungsloch (38) eingeführt wird.

6. Chirurgische Vorrichtung nach Anspruch 4, wobei das Führungsloch (38) einen Kanal definiert, der zu dem inneren Durchgang (24) der Einführkanüle (12) konzentrisch positioniert ist, wobei der Kanal konfiguriert ist, um die Ablationssonde (50) durch den inneren Durchgang (24) derart zu leiten, dass die Einführkanüle (12) und die Ablationssonde (50) durch einen Luftspalt (60) voneinander beabstandet sind.

7. Chirurgische Vorrichtung nach Anspruch 1, wobei das chirurgische Werkzeug eine Ablationssonde (50) ist und wobei das Abdichtungselement (30) über eine gummierte Reibpassung an der Einführkanüle (12) befestigt ist.

8. Chirurgisches System (10) zur Verwendung bei einem minimalinvasiven chirurgischen Eingriff, wobei das System eine chirurgische Vorrichtung nach Anspruch 1 und ein chirurgisches Werkzeug umfasst.

9. Chirurgisches System (10) nach Anspruch 8, wobei das chirurgische Werkzeug ein elastisches Element (52) umfasst, das bemessen ist, um den inneren Durchgang (24) der Einführkanüle (12) zu berühren.

10. Chirurgisches System (10) nach Anspruch 8, wobei das chirurgische Werkzeug eine chirurgische Nadel, einen HF-Stimulator oder eine Kryoablationssonde umfasst.

11. Chirurgisches System (10) nach Anspruch 8, wobei die distale Spitze (16) eine Öffnung umfasst, um den Durchgang eines therapeutischen Mittels zu einer Operationsstelle zu ermöglichen.

12. Chirurgisches System nach Anspruch 8, wobei das chirurgische Werkzeug eine Ablationssonde (50) ist, wobei die fluiddichte Oberfläche (36) ein Führungsloch (38) umfasst, das einen Kanal definiert, der zu dem inneren Durchgang (24) der Einführkanüle (12) konzentrisch positioniert ist, wobei der Kanal konfiguriert ist, um die Ablationssonde (50) durch den inneren Durchgang (24) derart zu leiten, dass die Einführkanüle (12) und die Ablationssonde (50) durch einen Luftspalt (60) voneinander beabstandet sind.

13. Chirurgisches System nach Anspruch 12, wobei das Abdichtungselement (30) elastisch ist und das Führungsloch (38) zwischen einer ersten Konfiguration und einer zweiten Konfiguration bewegbar ist, wobei die erste Konfiguration ein kleinerer Durchmesser ist und bemessen ist, um Fluid daran zu hindern, in die Einführkanüle (12) einzutreten, und die zweite Konfiguration ein größerer Durchmesser als die erste Konfiguration ist und bemessen ist, um die Ablationssonde (50) aufzunehmen.

14. Chirurgisches System nach Anspruch 8, wobei das chirurgische Werkzeug innerhalb des inneren Durchgangs (24) der Einführkanüle (12) konzentrisch positioniert ist, wobei der innere Durchgang (24) konfiguriert ist, um das chirurgische Werkzeug durch den inneren Durchgang (24) derart zu leiten, dass die Einführkanüle (12) und das chirurgische Werkzeug durch einen Luftspalt (60) voneinander beabstandet sind.

15. Chirurgisches System nach Anspruch 8, wobei das Abdichtungselement (30) über eine gummierte Reibpassung an der Einführkanüle (12) befestigt ist.

16. Chirurgisches System nach Anspruch 8, wobei die Einführkanüle (12) eine Öffnung an einem distalen Ende umfasst, die konfiguriert ist, um dem chirurgischen Werkzeug zu ermöglichen, sich durch die Öffnung zu erstrecken, um Gewebe angrenzend an die Öffnung zu schneiden.

## Revendications

1. Dispositif chirurgical comprenant une canule d'insertion (12) qui comprend un tube (14) ayant une extrémité proximale (18), une surface extérieure (20) comprenant une pointe distale (16) conçue pour pénétrer dans le tissu, et une surface intérieure (22) qui définit un passage interne (24) de la canule d'insertion (12), le passage interne (24) s'étendant le long d'un axe longitudinal (L) de la canule d'insertion (12) et conçu pour recevoir un outil chirurgical, un élément d'étanchéité (30) en contact avec l'extrémité proximale (18) du tube (14) et conçu pour fournir un joint d'étanchéité afin d'empêcher le fluide d'entrer ou de sortir de l'extrémité proximale (18) du tube (14) ;
l'élément d'étanchéité (30) comportant un corps évasé (32) s'étendant entre une extrémité distale (40) et une extrémité proximale (42) ;
le corps évasé (32) définissant quatre côtés évasés de sorte que la forme en coupe transversale de l'élément d'étanchéité (30) vers l'extrémité proximale (42) est un quadrilatère, les quatre côtés évasés étant plus larges à l'extrémité proximale (42) qu'à l'extrémité distale (40) ;
la forme évasée du corps (32) définissant une cavité intérieure (34), la cavité (34) s'étendant de manière distale depuis l'extrémité proximale (42) et se terminant au niveau d'une surface étanche aux fluides (36), la cavité (34) se rétrécissant vers la surface étanche aux fluides (36) ;
la surface étanche aux fluides (36) étant conçue pour venir en butée contre le tube (14) à l'extrémité proximale (18) et pour empêcher le fluide dans le passage interne (24) ; et
la surface étanche aux fluides (36) comprenant un matériau élastique et étant déformable pour permettre le passage de l'outil chirurgical à travers la surface (36) dans le passage interne (24).

2. Dispositif chirurgical selon la revendication 1, l'outil chirurgical étant une sonde d'ablation (50), et la canule d'insertion (12) comprenant une ouverture à une extrémité distale conçue pour permettre à la sonde d'ablation (50) de s'étendre à travers l'ouverture. pour réaliser l'ablation du tissu adjacent à l'ouverture.

3. Dispositif chirurgical selon la revendication 1, l'outil chirurgical étant une sonde d'ablation (50), et l'élément d'étanchéité (30) étant aligné sur le passage interne (24) et une surface externe (54) de la sonde d'ablation (50) ayant un diamètre plus petit qu'un diamètre de la surface intérieure (22) de la canule d'insertion (12) de sorte que lorsqu'une partie de la sonde d'ablation (50) se trouve à l'intérieur de la canule d'insertion (12), un intervalle d'air (60) est fourni dans le passage interne (24) circonférentiellement autour de la sonde d'ablation (50).

4. Dispositif chirurgical selon la revendication 1, l'outil chirurgical étant une sonde d'ablation (50), et la surface étanche aux fluides (36) comprenant un trou d'implantation (38) conçu pour recevoir la sonde d'ablation (50).

5. Dispositif chirurgical selon la revendication 4, l'élément d'étanchéité (30) étant élastique et le trou d'implantation (38) étant mobile entre une première configuration et une seconde configuration, la première configuration étant dimensionnée pour empêcher le fluide d'entrer dans la canule d'insertion (12) et la seconde configuration étant dimensionnée pour recevoir la sonde d'ablation (50), éventuellement,
(i) le trou d'implantation (38) comprenant un diamètre plus grand lorsqu'il est dans la seconde configuration que lorsqu'il est dans la première configuration, et/ou
(ii) le trou d'implantation (38) étant sollicité dans la première configuration pour fournir un ajustement par frottement étanche à l'air pour la sonde d'ablation (50) insérée à travers le trou d'implantation (38).

6. Dispositif chirurgical selon la revendication 4, le trou d'implantation (38) définissant un canal positionné de manière concentrique avec le passage interne (24) de la canule d'insertion (12), le canal étant conçu pour guider la sonde d'ablation (50) à travers le passage interne (24) de sorte que la canule d'insertion (12) et la sonde d'ablation (50) sont espacées par un intervalle d'air (60).

7. Dispositif chirurgical selon la revendication 1, l'outil chirurgical étant une sonde d'ablation (50), et l'élément d'étanchéité (30) étant fixé à la canule d'insertion (12) par le biais d'un ajustement par frottement caoutchouté.

8. Système chirurgical (10) destiné à être utilisé dans une intervention chirurgicale minimalement invasive, le système comprenant un dispositif chirurgical selon la revendication 1 et un outil chirurgical.

9. Système chirurgical (10) selon la revendication 8, l'outil chirurgical comprenant un élément élastique (52) dimensionné pour entrer en contact avec le passage interne (24) de la canule d'insertion (12).

10. Système chirurgical (10) selon la revendication 8, l'outil chirurgical comprenant une aiguille chirurgicale, un stimulateur RF ou une sonde de cryo-ablation.

11. Système chirurgical (10) selon la revendication 8, la pointe distale (16) comprenant une ouverture pour permettre le passage d'un agent thérapeutique vers un champ opératoire.

12. Système chirurgical selon la revendication 8, l'outil chirurgical étant une sonde d'ablation (50), la surface étanche aux fluides (36) comprenant un trou d'implantation (38) qui définit un canal positionné de manière concentrique avec le passage interne (24) de la canule d'insertion (12), le canal étant conçu pour guider la sonde d'ablation (50) à travers le passage interne (24) de sorte que la canule d'insertion (12) et la sonde d'ablation (50) sont espacées par un intervalle d'air (60).

13. Système chirurgical selon la revendication 12, l'élément d'étanchéité (30) étant élastique et le trou d'implantation (38) étant mobile entre une première configuration et une seconde configuration, la première configuration étant d'un diamètre plus petit et dimensionnée pour empêcher le fluide d'entrer dans la canule d'insertion (12) et la seconde configuration étant d'un diamètre plus grand que la première configuration et dimensionnée pour recevoir la sonde d'ablation (50).

14. Système chirurgical selon la revendication 8, l'outil chirurgical étant positionné de manière concentrique à l'intérieur du passage interne (24) de la canule d'insertion (12), le passage interne (24) étant conçu pour guider l'outil chirurgical à travers le passage interne (24) de sorte que la canule d'insertion (12) et l'outil chirurgical sont espacés par un intervalle d'air (60).

15. Système chirurgical selon la revendication 8, l'élément d'étanchéité (30) étant fixé à la canule d'insertion (12) par le biais d'un ajustement par frottement caoutchouté.

16. Système chirurgical selon la revendication 8, la canule d'insertion (12) comprenant une ouverture à une extrémité distale conçue pour permettre à l'outil chirurgical de s'étendre à travers l'ouverture pour couper le tissu adjacent à l'ouverture.
